# EUROPEAN PATENT APPLICATION

(11) **EP 4 303 644 A1**
(43) Date of publication of application: **10.01.2024**
(21) Application number: 22306009.6
(22) Date of filing: 06.07.2022
(51) Int. Cl.: G02C 7/10, G01J 3/46, G01J 3/52, A61B 5/103

(54) **METHOD FOR PROVIDING AN EYEGLASS HAVING A TINT SELECTED AMONG A PLURALITY OF PREDETERMINED EYEGLASS TINTS AND HAND TOOL USED IN A METHOD FOR PROVIDING AN EYEGLASS HAVING A TINT SELECTED AMONG A PLURALITY OF PREDETERMINED EYEGLASS TINTS**

(71) Applicant: Essilor International, 94220 Charenton-Le-Pont (FR)
(72) Inventor: ANG, Ker Chin, S39346 Singapore (SG)
(74) Representative: Santarelli

(57) **Abstract**

The disclosure relates to a method for providing an eyeglass having a tint selected among a plurality of tints, including the steps of providing a hand tool; placing said hand tool close to a region of interest on the individual's skin; determining the skin tone of a skin tones palette being the closest to said individual's skin tone; identifying a marker for a preferred tint in said plurality of tints from the determined skin tone, thanks to a graphical correspondence on the hand tool; and to a hand tool used in such method, comprising: a panel; a palette of skin tones arranged along an edge; markers for tints arranged along said skin tones palette; each skin tone of said skin tones palette being in graphical correspondence with a marker for a tint, according to a predetermined relationship which defines, for each skin tone, a preferred tint.

## Description

The disclosure relates to a method for providing an eyeglass having a tint selected among a plurality of predetermined eyeglass tints, the selected tint depending on an individual's skin tone in a region of interest on the individual's skin.

The disclosure also relates to a hand tool used in a method for providing an eyeglass having a tint selected among a plurality of predetermined eyeglass tints.

The disclosure also relates to a kit comprising a hand tool used in a method for providing an eyeglass having a tint selected among a plurality of predetermined eyeglass tints, and a set of eyeglasses each having a tint belonging the plurality of eyeglass tints.

### BACKGROUND ART

Methods and tools for enhancing the physical appearance of a wearer are known from the prior art. A known method solution consists of selecting a tinted eyeglass from a subset of eyeglass tints that are suitable for a given skin tone base colour. The step of determining the skin tone base colour can be uncertain. Another known solution consists of conducting an assessment comprising several questions, so as to determine the most suitable eyeglass tints for an individual attending the assessment. The questions and the responses can be subjective.

### SUMMARY OF THE DISCLOSURE

The disclosure is directed to a method for providing an eyeglass having a tint selected among a plurality of predetermined eyeglass tints, which is easy, accurate and convenient to implement.

The disclosure accordingly provides, according to a first aspect, a method for providing an eyeglass having a tint selected among a plurality of predetermined eyeglass tints, the selected tint depending on an individual's skin tone in a region of interest on the individual's skin, including the steps of:
- providing a hand tool for identifying the tint of an eyeglass from the individual's skin tone, based on a previously established relationship between the plurality of skin tones and a plurality of markers for eyeglass tints, said tool comprising a panel, a palette of said skin tones arranged along an edge of said panel and said markers of eyeglass tints arranged along said skin tones palette on an opposite side to said edge, each skin tone of said skin tones palette being in graphical correspondence with at least one marker for an eyeglass tint of said eyeglass tints palette according to said previously provided relationship;
- placing said edge of said hand tool close to said region of interest on the individual's skin;
- determining the skin tone of said skin tones palette which is the closest to said individual's skin tone in the region of interest;
   identifying at least one marker for a preferred eyeglass tint in said plurality of eyeglass tints from the determined skin tone, thanks to the graphical correspondence on the hand tool.

According to the disclosure, the method achieves a selection of an eyeglass with the most suitable tint with regard to an individual's skin tone in a region of interest, only by very simple and objective steps carried out thanks to the hand tool.

In particular, the hand tool provided in this method allows to directly determine a closest skin tone to the actual skin tone of the individual and next, to select the most suitable eyeglass tint from the determined skin tone.

Providing the hand-tool allows to make a direct and practical use of the previously determined relationship, which is thus made accessible to any user of the tool.

In addition, such a hand-tool is easy to provide and to handle.

Compared to the more subjective known methods, this avoids any bias which would be induced if the skin tone is estimated or guessed. Overall, the recommendation of an eyeglass tint for an individual with a given skin tone is more accurate and personalized.

Advantageous and convenient features of the method are described below.

The previously established relationship defines, for each skin tone of a plurality of skin tones, at least one preferred eyeglass tint of the plurality of predetermined eyeglass tints which enhances the physical appearance of an individual having a skin tone corresponding to said each skin tone.

The graphical correspondence between each skin tone of the skin tones palette and at least one marker for an eyeglass tint of the plurality of predetermined eyeglass tints is the alignment of said skin tone and said at least one marker for an eyeglass tint along a direction which is locally transversal to said edge.

At least one skin tone of said skin tones palette is in graphical correspondence with at least two of said markers and/or at least one marker for an eyeglass tint of the plurality of predetermined eyeglass tints is in graphical correspondence with at least two skin tones of said skin tones palette.

At least two markers, respectively the at least two skin tones, are each graphically at different distances from the graphically corresponding at least one skin tone, respectively the graphically corresponding at least one marker for an eyeglass tint, on the hand tool.

The step of determining the skin tone and/or the step of identifying at least one marker for a preferred eyeglass tint is carried out depending on the different distances between the at least two markers, respectively the at least two skin tones, from the graphically corresponding at least one skin tone, respectively the graphically corresponding at least one marker for an eyeglass tint, on the hand tool.

Each skin tone of said skin tones palette is a graphical block on the panel and each marker for an eyeglass tint is a strip on the panel having locally the same orientation as the edge and at least one such strip extends over multiple skin tone graphical blocks.

Each skin tone of said skin tone palette is a skin tone gradient strip on the panel, having locally the same orientation as the edge, and each marker for an eyeglass tint is a strip on the panel having locally the same orientation as the edge, the strips extending at least partially over each other.

The method further comprises the step of selecting at least one eyeglass having a tint corresponding to said at least one identified preferred eyeglass tint in a set of eyeglasses each having a different tint belonging to the plurality of eyeglass tints.

The disclosure also provides, according to a second aspect, a hand tool used in a method for providing an eyeglass having a tint selected among a plurality of predetermined eyeglass tints, the selected tint depending on an individual's skin tone in a region of interest on the individual's skin; the hand tool comprising:
- a panel;
- a palette of skin tones being arranged along an edge of said panel;
- markers of said predetermined eyeglass tints being arranged along said skin tones palette on an opposite side to said edge;
each skin tone of said skin tones palette being in graphical correspondence with at least one marker for an eyeglass tint of said plurality of eyeglass tints, according to a previously determined relationship which defines, for each skin tone of a plurality of skin tones, at least one preferred eyeglass tint of the plurality of predetermined eyeglass tints which enhances the physical appearance of an individual having a skin tone corresponding to said each skin tone; and
the hand tool being configured so that the skin tone of said skin tones palette which is the closest to the individual's skin tone in the region of interest on the individual's skin is determined by placing said edge of said hand tool close to said region of interest on the individual's skin, and the marker for an eyeglass tint corresponding to at least one preferred eyeglass tint identified among the plurality of eyeglass tints is selected from the determined closest skin tone.

According to the disclosure, the hand tool allows to directly determine a closest skin tone to the actual skin tone of the individual and next, to select the most suitable eyeglass tint from the determined skin tone.

The hand-tool allows to make a direct and practical use of the previously determined relationship, which is thus made accessible to any user of the tool.

In addition, such a hand-tool is easy to provide and to handle.

The hand tool can be used for carrying out simple and objective steps so as to achieve a selection of an eyeglass with the most suitable tint with regard to an individual's skin tone in a region of interest.

Overall, the hand tool allows to avoid any bias which would be induced if the skin tone is estimated or guessed for recommending a suitable eyeglass tint. The recommendation of an eyeglass tint for an individual with a given skin tone is more accurate and personalized thanks to the disclosed hand tool.

Advantageous and convenient features of the hand tool are described below.

The graphical correspondence between each skin tone and at least one eyeglass tint is the alignment of said skin tone and said at least one marker for an eyeglass tint along a direction which is locally transversal to said edge.

The panel has a generally rectangular outline and said edge along which said skin tones palette is arranged, forms a longitudinal side of said outline.

The panel has a longitudinal extension generally corresponding to the extension of the human eye according to a nasal-temporal direction and said edge is concave for matching the general curvature of a side of a human eyelid.

The panel is annular and said edge delimits a central hole of said annular panel, said panel having an outer diameter between 10 mm and 50 mm.

The disclosure further provides, according to a third aspect, a kit comprising a hand tool and a set of eyeglasses each having a tint belonging the plurality of eyeglass tints.

### BRIEF DESCRIPTION OF THE DRAWINGS

The description of the disclosure now continues with a detailed description of advantageous embodiments given hereinafter by way of nonlimiting example and with reference to the appended drawings.
Figure 1 schematically depicts a hand tool according to a first embodiment, which is used in a method for providing an eyeglass having a tint selected among a plurality of predetermined eyeglass tints.
Figure 2 schematically depicts a hand tool according to a second embodiment, which is also used in the method for providing an eyeglass having a tint selected among a plurality of predetermined eyeglass tints.
Figure 3 schematically depicts a hand tool according to a third embodiment, which is also used in such a method.
Figure 4 is a block diagram illustrating operating steps of the method.

### DETAILED DESCRIPTION OF EXAMPLE EMBODIMENTS

In the following description, similar features in different embodiments are referred to with the same reference numbers.

The disclosure is directed to a hand tool used in a method for providing an eyeglass having a tint selected among a plurality of predetermined eyeglass tints, and to such a method.

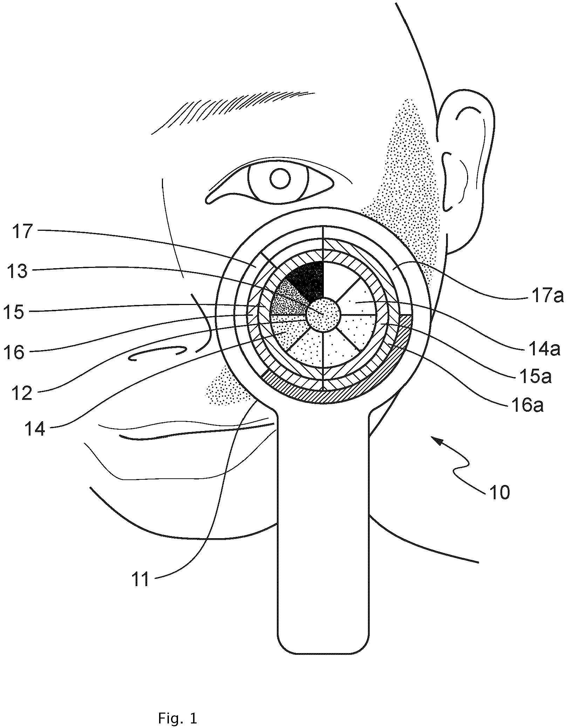

Figure 1 illustrates such a hand tool 10 according to a first embodiment.

The hand tool 10 is configured to be used in a method for providing an eyeglass having a tint selected among a plurality of predetermined eyeglass tints.

The hand tool 10 comprises a panel 11.

The panel 11 has here a shape of an annular disc.

The diameter of the panel 11 is comprised between 10 mm and 50 mm, and is preferably of around 20 mm.

The panel 11 can be made of cardboard, plastic sheet or any other suitable material.

For convenient holding, the hand tool 10 may further comprise a handle extending from an outline of the panel 11.

The panel 11 has an inner edge 12, which delimits a central hole 13 of the annular disc.

The hand tool 10 comprises a skin tones palette 14, which is arranged along the inner edge 12 of the panel 11.

In Figure 1, the skin tones palette 14 comprises a plurality of graphical blocks 14a arranged side by side along the inner edge 12.

In other words, the graphical blocks 14a here form rings or ring sections on the panel 11.

Each graphical block 14a has a different colour, which corresponds to predetermined skin tones.

For example, the colours range from a nearly white colour to a dark brown colour and comprise different nuances of beige and brown.

In variant, the skin tones palette 14 may comprise a skin tones gradient strip which has locally the same orientation as the inner edge.

The hand tool 10 further comprises markers for eyeglass tints.

In particular, in Figure 1, the hand tool 10 comprises first markers 15 for a dedicated eyeglass tint.

The first markers 15 are here arranged along the skin tones palette 14 on an opposite side to the inner edge 12.

The first markers 15 comprise a plurality of strips 15a, which are arranged end by end along the skin tones palette 14.

The strips 15a have locally the same orientation as the inner edge 12. In other words, each strip 15a here forms a ring or a ring section on the panel 11.

In Figure 1, the hand tool 10 further comprises second markers 16 dedicated for another eyeglass tint.

The second markers 16 are arranged along the first markers 15, on an opposite side to the inner edge 12.

In other words, the second markers 16 are farther away from the skin tone palette 14 than the first markers 15 are.

In Figure 1, the hand tool 10 further comprises third markers 17 for again another eyeglass tint, which are arranged along the second markers 16, on the opposite side to the inner edge 12.

In particular, the third markers 17 are farther away from the skin tone palette 14 than the second markers 16 are, and even farther away than the first markers 15 are.

The second markers 16 and third markers 17 have shapes similar to the first markers 15 and may comprise one or more strips 16a, 17a.

In Figure 1, the strips 15a, 16a, 17a are in graphical correspondence with a plurality of graphical blocks 14a.

In variant, at least one strip 15a, 16a, 17a can be in graphical correspondence with only a single graphical block 14a.

In another variant, at least one strip 15a, 16a, 17a can be in graphical correspondence with only a part of one or a plurality of graphical blocks 14a, so that one or a plurality of strips 15a, 16a, 17a may be in graphical correspondence with a single graphical block 14a.

It should be noted that at least one marker for an eyeglass tint can be a colour reminiscent of the eyeglass tint, or a colour corresponding exactly to the eyeglass tint.

In variant, other markers can be chosen, for example, a marker for an eyeglass tint can be a reference code for the eyeglass tint.

As explained above, the skin tones of the skin tones palette 14 and at least one marker for an eyeglass tint are in graphical correspondence on the panel 10.

The graphical correspondence is the arrangement of a skin tone and at least one marker for an eyeglass tint along a direction which has locally the same orientation as the inner edge 12.

Some directions are represented as a dotted line across the graphical block 14a in Figure 1.

In other words, the arrangement wherein a graphical correspondence can be identified is a radial section of the panel 10, extending from the inner edge 12 to its opposite edge.

For instance, in Figure 1, the strip 15a of the first marker 15, the strip 16a of the second marker 16 and the strip 17a of the third marker 17 are in graphical correspondence with the graphical block 14a.

In addition, such an arrangement showing a graphical correspondence may define an order of preference of the eyeglass tint, for each skin tone which is graphical correspondence, from the skin tones palette 14.

For instance, the closer to the skin tones palette 14 the marker for an eyeglass tint is, the more it can be preferred, between the markers in correspondence to a given skin tone graphical block.

It is to be noted that the graphical correspondence between a skin tone of the skin tones palette 14 and one or more marker for an eyeglass tint is given by a previously determined relationship.

This relationship defines, for each skin tone of a plurality of skin tones, at least one preferred eyeglass tint of the plurality of predetermined eyeglass tints which enhances the physical appearance of an individual having a skin tone corresponding to said each skin tone.

A relationship between skin tone and a recommended tint is established for a population.

For instance, for a population whose skin tone ranged between light to dark, the preferred tints can be either light pink or light purple hues; while for a population with light to intermediate skin tone, light pink tinted lenses can be preferred.

One can note that for light to intermediate skin tones, two hues may have an effect. The hue having, according to the determined relationship, more chances of enhancing the physical appearance of the wearer is the closer it is positioned to the skin tone palette.

Such a relationship is established so as to be representative of a generally accepted beauty enhancing effect by observation by a plurality of trained neutral observers.

The trained neutral observers may then provide a notation about a plurality of tinted lenses with different hues and the perception of beauty enhancing effect when said lens are worn by different wearers having a variety of skin tones.

By collecting those data, a relationship between some hues and some skin tones, some tinted lenses having a beauty enhancing effect when worn by some people of specific skin tones, may be identified.

Such a relationship obtained can be seen as a social-scientific study and allows to identify trends in terms of reflected beauty in society.

It is also possible to establish different relationships for different geographical areas, since the perceived beauty of an individual may depend from social and geographical parameters.

The graphical correspondences between the skin tones of the skin tones palette and the markers for eyeglass tints illustrates this relationship, which thus becomes directly usable.

The hand tool 10 is used as follow.

The hand tool 10 is configured to be placed close to a region of interest on an individual's skin.

In particular, the hand tool 10 of Figure 1 is situated with respect to the individual' skin so that the central hole 13 is located on the region of interest, namely the region of the cheeks, or close to it.

The skin tone of the skin tones palette 14 which is the closest to the individual's skin tone in the region of interest can thus be determined.

Next, at least one of the first, second and/or third markers 15, 16, 17 for eyeglass tints in graphical correspondence with the determined closest skin tone can be identified thanks to the hand tool 10.

Depending of the optional order of preference of the first, second and/or third markers 15, 16, 17, at least an eyeglass tint can be selected for the individual.

Since several eyeglass tints can suit to an individual, one or several other eyeglass tints can be recommended, with an order of preference if any.

For instance, on the hand tool 10 illustrated in Figure 1, the most preferred eyeglass tint can be defined by the first marker 15, a second preferred eyeglass tint can be defined by the second marker 16, and a third preferred eyeglass tint can be defined by the third marker 17.

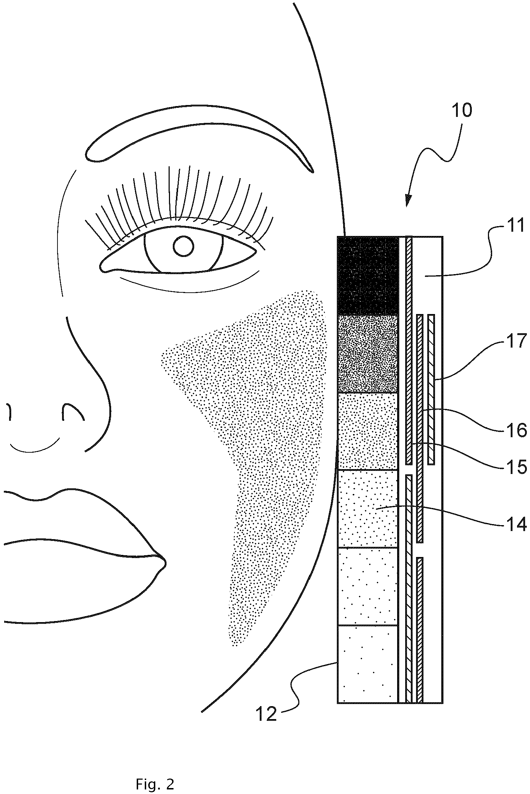

Figure 2 illustrates a second embodiment of the hand tool 10, which also comprises a panel 11 having here a rectangular outline rather than an annular disc shape.

The hand tool 10 comprises a skin tones palette 14 which is arranged along a first longitudinal, inner edge 12 of the rectangular outline.

The skin tones palette 14 comprises several graphical blocks 14a arranged side by side.

In other words, the skin tones palette 14 here forms a longitudinal strip along the panel 10.

In variant, the skin tones palette 14 can also be a skin tones gradient strip, similar to the variant described in the first embodiment.

Accordingly, first, second and/or third markers 15, 16, 17 for eyeglass tints comprise one or more strips arranged adjacent to the skin tones palette 14.

More generally, the variant mentioned in reference to the hand tool shown on Figure 1 also applies to the hand tool of Figure 2.

The hand tool 10 is used as follow.

The hand tool 10 is configured to be placed close to the region of interest on an individual's skin.

In particular, the hand tool 10 of Figure 2 is situated with respect to the individual' skin so that the first longitudinal edge 12 of the rectangular outline is located on the region of interest, namely the region of the cheeks, or close to it.

The skin tone of the skin tones palette 14 which is the closest to the individual's skin tone in the region of interest can thus be determined, optionally by displacing the hand tool 10.

Next, at least one of the first, second and/or third markers 15, 16, 17 for eyeglass tints in graphical correspondence with the determined closest skin tone can be identified thanks to the hand tool 10.

Depending of the optional order of preference of the first, second and/or third markers 15, 16, 17, at least an eyeglass tint can be selected for the individual.

Since several eyeglass tints can suit to an individual, one or several other eyeglass tints can be recommended, with an order of preference if any.

For instance, on the hand tool 10 illustrated in Figure 2, the most preferred eyeglass tint according to the predetermined relationship can be defined by the first marker 15, optionally a second preferred eyeglass tint can be defined by the second marker 16, and optionally a third preferred eyeglass tint can be defined by the third marker 17.

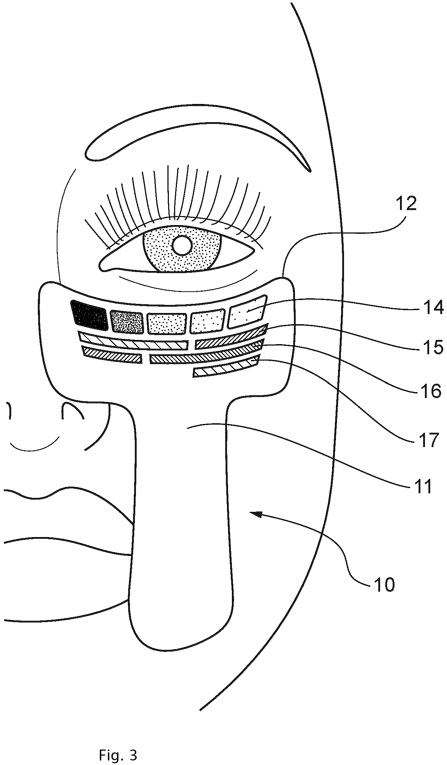

Figure 3 illustrates a third embodiment of the hand tool 10, which also comprises a panel 11 having here a shape of a brush rather than an rectangular outline and an annular disc shape.

In particular, the panel 11 has a longitudinal portion generally corresponding to the extension of the human eye according to a nasal-temporal direction, and the hand tool 10 has a handle portion extending below the longitudinal portion.

The hand tool 10 has a skin tones palette 14 which is arranged along an inner edge 12 which is here concave. The skin tones palette 14 here comprises graphical blocks.

The hand tool 10 has also markers 15, 16, 17 for eyeglass tints as disclosed above. The markers 15, 16, 17 here comprises longitudinal strips.

The hand tool 10 shown on Figure 3 is thus located more closer to the eye of the individual than the hand tools shown on Figures 1 and 2.

The hand tool 10 is used as follow.

The hand tool 10 is configured to be placed under the eye and in particular in the region of interest on an individual's skin.

In particular, the hand tool 10 of Figure 3 is situated with respect to the individual' skin so that the inner edge 12 which is concave is located on the region of interest, namely the region of the dark circle under the eye.

The skin tone of the skin tones palette 14 which is the closest to the individual's skin tone in the region of interest can thus be determined.

Next, at least one of the first, second and/or third markers 15, 16, 17 for eyeglass tints in graphical correspondence with the determined closest skin tone can be identified thanks to the hand tool 10.

Depending of the optional order of preference of the first, second and/or third markers 15, 16, 17, at least an eyeglass tint can be selected for the individual.

Since several eyeglass tints can suit to an individual, one or several other eyeglass tints can be recommended, with an order of preference if any.

For instance, on the hand tool 10 illustrated in Figure 3, the most preferred eyeglass tint according to the predetermined relationship can be defined by the first marker 15, optionally a second preferred eyeglass tint can be defined by the second marker 16, and optionally a third preferred eyeglass tint can be defined by the third marker 17.

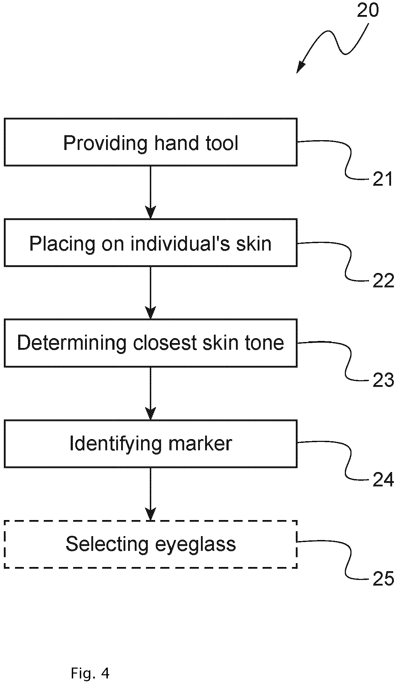

Figure 4 illustrates more generally a method 20 for providing an eyeglass having a tint selected among a plurality of predetermined eyeglass tints.

The method 20 comprises the step 21 of providing a hand tool 10 for identifying the tint of an eyeglass from an individual's skin tone, based on the previously established relationship between the plurality of skin tones and the plurality of markers 15, 16, 17 for an eyeglass tints.

As described above, the hand tool 10 comprises a panel 11, a palette of skin tones, or skin tones palette 14, arranged along an edge 12 of the panel 11 and markers 15, 16, 17 of eyeglass tints arranged along the skin tones palette 14 on an opposite side to the edge 12.

Each skin tone of said skin tones palette 14 is in graphical correspondence with at least one marker for an eyeglass tint of said plurality of eyeglass tints according to said previously provided relationship.

The method 20 further comprises the step 22 of placing the edge 12 of the hand tool 10 close to a region of interest on the individual's skin.

The method 20 further comprises the step 23 of determining the skin tone of the skin tones palette 14 which is the closest to the individual's skin tone in the region of interest thanks to the hand tool.

The method 20 further comprises the step 24 of identifying at least one marker for a preferred eyeglass tint in the plurality of eyeglass tints palette 14 from the determined skin tone, thanks to the graphical correspondence on the hand tool 10.

The hand tool 10 provided in method step 21 may be one of the hand tools described above in reference to Figures 1 to 3.

The graphical correspondences between the skin tones palette 14 and the markers 15, 16, 17 is obtained from the previously provided relationship.

The previously established relationship defines, for each skin tone of a plurality of skin tones, at least one preferred eyeglass tint of the plurality of predetermined eyeglass tints which enhances the physical appearance of an individual having a skin tone corresponding to said each skin tone.

As described hereabove, such a relationship can be obtain empirically by collecting data from objective human observers.

For example, the observers rate the beauty enhancing effect of a given eyeglass tint for a given skin tone by observing a panel of individuals with different skin tones, wearing eyeglasses of different tints from the plurality of eyeglass tints.

More specifically, the observers may rank the eyeglass tints from a most suitable eyeglass tint to a least suitable eyeglass tint, for a given skin tone. This allows to establish for instance an order of preference of the eyeglass tints for a given skin tone.

As described hereabove, the order of preference can be expressed on the hand tool 10 by representing more than one marker for an eyeglass tint on the panel 11. In particular, the farther away from the skin tones palette 14 the markers are, the least preferred they are. Inversely, the markers the closest to the skin tones palette 14 are preferred.

In step 22, the edge 12 of the panel 10 can be located the cheeks of the dark circles under eyes of the individual, where the eyeglass will have a beauty enhancing effect.

In step 23, the determination of the skin tone of the skin tones palette 14 which is the closest to the skin tone of the individual's skin in the region of interest can be done visually, by eye.

In step 24, the identification at least one marker for an eyeglass tint is made thanks to the graphical correspondence, starting from the closest skin tone determined in step 23. In particular, the graphical correspondence allows to identify at least one first marker 15 which is arranged along the skin tones palette 14. If the hand tool 10 comprises further markers like second markers 16 and a third markers 17, at least one of them can also be identified.

Based on step 24, at least one eye tint can be selected.

Optionally, the method further comprises a step 25 of selecting at least one eyeglass having a tint corresponding to said at least one selected preferred eyeglass tint in a set of eyeglasses each having a different tint belonging to the plurality of eyeglass tints.

Accordingly, a spectacle having eyeglasses with the selected preferred tint can be provided to the individual.

For instance, an optician may have a kit with a hand tool 10 as described above and a set of eyeglasses each having a tint belonging the plurality of eyeglass tints is provided, in a way that the hand tool 10 matches to the eyeglass tints that are available.

It should be noted more generally that the disclosure is not limited to the examples described and represented.

## Claims

1. Method for providing an eyeglass having a tint selected among a plurality of predetermined eyeglass tints, the selected tint depending on an individual's skin tone in a region of interest on the individual's skin, including the steps of:
- providing (21) a hand tool (10) for identifying the tint of an eyeglass from the individual's skin tone, based on a previously established relationship between the plurality of skin tones and a plurality of markers for eyeglass tints, said tool comprising a panel (11), a palette of said skin tones (14) arranged along an edge (12) of said panel and said markers (15, 16, 17) of eyeglass tints arranged along said skin tones palette on an opposite side to said edge (12), each skin tone of said skin tones palette being in graphical correspondence with at least one marker for an eyeglass tint of said eyeglass tints palette according to said previously provided relationship;
- placing (22) said edge (12) of said hand tool (10) close to said region of interest on the individual's skin;
- determining (23) the skin tone of said skin tones palette (14) which is the closest to said individual's skin tone in the region of interest;
- identifying (24) at least one marker (15, 16, 17) for a preferred eyeglass tint in said plurality of eyeglass tints from the determined skin tone, thanks to the graphical correspondence on the hand tool (10).

2. Method according to claim 1, wherein the previously established relationship defines, for each skin tone of a plurality of skin tones, at least one preferred eyeglass tint of the plurality of predetermined eyeglass tints which enhances the physical appearance of an individual having a skin tone corresponding to said each skin tone.

3. Method according to any of claims 1 and 2, wherein said graphical correspondence between each skin tone of the skin tones palette (14) and at least one marker (15, 16, 17) for an eyeglass tint of the plurality of predetermined eyeglass tints is the alignment of said skin tone and said at least one marker (15, 16, 17) for an eyeglass tint along a direction which is locally transversal to said edge (12).

4. Method according to any of claims 1 to 3, wherein at least one skin tone of said skin tones palette (14) is in graphical correspondence with at least two markers (15, 16, 17) for an eyeglass tint of the plurality of predetermined eyeglass tints and/or at least one marker (15, 16, 17) for an eyeglass tint of the plurality of predetermined eyeglass tints is in graphical correspondence with at least two skin tones of said skin tones palette (14).

5. Method according to claim 4, wherein the at least two markers (15, 16, 17), respectively the at least two skin tones, are each graphically at different distances from the graphically corresponding at least one skin tone, respectively the graphically corresponding at least one marker (15, 16, 17) for an eyeglass tint, on the hand tool (10).

6. Method according to claim 5, wherein the step of determining (23) the skin tone and/or the step of identifying (24) at least one marker for a preferred eyeglass tint is carried out depending on the different distances between the at least two markers (15, 16, 17), respectively the at least two skin tones, from the graphically corresponding at least one skin tone, respectively the graphically corresponding at least one marker (15, 16, 17) for an eyeglass tint, on the hand tool (10).

7. Method according to any of claims 4 to 6, wherein each skin tone of said skin tones palette (14) is a graphical block (14a) on the panel (11) and each marker (15, 16, 17) for an eyeglass tint is a strip (15a, 16a, 17a) on the panel (11) having locally the same orientation as the edge (12) and at least one such strip (15a, 16a, 17a) extends over multiple skin tone graphical blocks (14a).

8. Method according to any of claims 4 to 6, wherein each skin tone of said skin tone palette (14) is a skin tone gradient strip on the panel, having locally the same orientation as the edge (12), and each marker (15, 16, 17) for an eyeglass tint is a strip (15a, 16a, 17a) on the panel having locally the same orientation as the edge (12), the strips extending at least partially over each other.

9. Method according to any of claims 1 to 8, further comprising the step of selecting (25) at least one eyeglass having a tint corresponding to said at least one identified preferred eyeglass tint in a set of eyeglasses each having a different tint belonging to the plurality of eyeglass tints.

10. Hand tool used in a method for providing an eyeglass having a tint selected among a plurality of predetermined eyeglass tints, the selected tint depending on an individual's skin tone in a region of interest on the individual's skin; the hand tool comprising:
- a panel (11);
- a palette of skin tones (14) being arranged along an edge (12) of said panel;
- markers (15, 16, 17) of said predetermined eyeglass tints being arranged along said skin tones palette (14) on an opposite side to said edge (12); each skin tone of said skin tones palette (14) being in graphical correspondence with at least one marker (15, 16, 17) for an eyeglass tint of said plurality of eyeglass tints, according to a previously determined relationship which defines, for each skin tone of a plurality of skin tones, at least one preferred eyeglass tint of the plurality of predetermined eyeglass tints which enhances the physical appearance of an individual having a skin tone corresponding to said each skin tone; and
the hand tool (10) being configured so that the skin tone of said skin tones palette (14) which is the closest to the individual's skin tone in the region of interest on the individual's skin is determined by placing said edge (12) of said hand tool (10) close to said region of interest on the individual's skin, and the marker (15, 16, 17) for an eyeglass tint corresponding to at least one preferred eyeglass tint identified among the plurality of eyeglass tints is selected from the determined closest skin tone.

11. Hand tool according to claim 10, wherein said graphical correspondence between each skin tone and at least one eyeglass tint is the alignment of said skin tone and said at least one marker for an eyeglass tint along a direction which is locally transversal to said edge (12).

12. Hand tool according to any of claims 10 to 11, wherein said panel (11) has a generally rectangular outline and said edge (12) along which said skin tones palette (14) is arranged, forms a longitudinal side of said outline.

13. Hand tool according to any of claims 10 or 11, wherein said panel (11) has a longitudinal extension generally corresponding to the extension of the human eye according to a nasal-temporal direction and said edge (12) is concave for matching the general curvature of a side of a human eyelid.

14. Hand tool according to any of claims 10 or 11, wherein said panel (11) is annular and said edge (12) delimits a central hole (13) of said annular panel, said panel (11) having an outer diameter between 10 mm and 50 mm.

15. Kit comprising a hand tool (10) according to any of claims 10 to 14 and a set of eyeglasses each having a tint belonging the plurality of eyeglass tints.
